# FASCICULE DE BREVET EUROPEEN

(11) **EP 1 351 727 B1**
(45) Date de publication et mention de la délivrance du brevet: **13.10.2004**
(21) Numéro de dépôt: 02700330.0
(22) Date de dépôt: 16.01.2002
(51) Int. Cl.: A61M 5/14, A61M 39/22, A61M 39/10

(54) **SET DE TRANSFERT NOTAMMENT POUR L'ADMINISTRATION D'UN MELANGE DE LIQUIDES A DES FINS MEDICALES**
VORRICHTUNG ZUM FLÜSSIGKEITSTRANSFER INSBESONDERE FÜR INFUSION EINER MISCHUNG VON FLÜSSIGKEITEN FÜR MEDIZINISCHE ZWECKE
TRANSFER SET IN PARTICULAR FOR DELIVERING A MIXTURE OF LIQUIDS FOR MEDICAL PURPOSES

(30) Priorité: 17.01.2001 FR 0100598
(43) Date de publication de la demande: 15.10.2003
(73) Titulaire: Technoflex S.A., 64210 Bidart (FR)
(72) Inventeur: CURUTCHARRY Jean, F-64210 Guethary (FR)
(74) Mandataire: Thébault, Jean-Louis
(86) Numéro de dépôt international: PCT/FR2002/000163
(87) Numéro de publication internationale: WO 2002/056946

(56) Documents cités:
- EP-A- 0 363 770
- WO-A-99/44652
- FR-A- 2 468 059
- US-A- 4 181 140
- US-A- 6 071 262

## Description

L'invention a trait plus particulièrement, bien que non exclusivement à l'administration à des fins médicales de substances liquides par exemple par voie parentérale, à partir d'un ensemble de poches souples comprenant divers constituants liquides à mélanger préalablement à l'administration.

Il est souvent nécessaire dans de telles administrations de réaliser auparavant, sur le site d'administration, des mélanges de quantités dosées par exemple d'un ou plusieurs principes actifs avec un diluant contenu dans une poche souple.

Il est alors nécessaire qu'un opérateur transfère manuellement les quantités requises de principes actifs, par exemple à l'aide d'une seringue dans la poche de diluant, avec tous les risques d'erreur d'une telle manipulation.

La présente invention vise à pallier ces divers inconvénients en proposant, un set de transfert permettant d'effectuer des combinaisons de principes actifs extrêmement variées dans des conditions de sécurité maximale aussi bien pour le patient que pour le personnel soignant.

A cet effet, l'invention a pour objet un set de transfert, notamment pour l'administration d'un mélange de liquides à des fins médicales, à partir d'une poche souple dite principale contenant un premier liquide et d'au moins une poche souple dite auxiliaire contenant un second liquide, caractérisé en ce que la poche auxiliaire est raccordable à la poche principale à l'aide d'une paire de connecteurs de type luer respectivement mâle et femelle, chacun des deux connecteurs comportant, à une extrémité, une section frangible engagée dans l'extrémité d'une tubulure reliée à l'une des poches et, à l'autre extrémité, un moyen d'accouplement à l'autre connecteur, pourvu de moyens de blocage en position accouplée desdits connecteurs.

Le dispositif de l'invention présente l'avantage de proposer des ensembles d'administration prêts à l'emploi, c'est à dire comprenant une poche principale par exemple de diluant pré-connectée, c'est à dire raccordée par une liaison tubulaire mais non communicante, à une poche dosée ou en parallèle à plusieurs poches dosées de principes actifs ou de nutriments, et dont la communication avec la poche de diluant peut être réalisée, in situ, à tout moment et instantanément par rupture des sections frangibles des luers de la communication à établir.

La pré-connection au sens défini ci-dessus est réalisée de manière particulièrement sûre du fait du verrouillage de l'accouplement de chaque paire de connecteurs, assurant ainsi une asepsie parfaitement contrôlée et évitant tout contact cutané ou aérien du personnel soignant avec des substances potentiellement très actives.

Une telle pré-connection peut bien entendu être réalisée à tout moment et notamment au moment de l'administration, en branchant sur une poche principale de diluant pourvue d'une ou plusieurs tubulures d'injection en parallèle équipées chacune d'un connecteur luer conforme à l'invention, une poche convenablement dosée pourvue d'une tubulure équipée d'un connecteur luer complémentaire qui est alors accouplé de manière irréversible au connecteur de l'une desdites tubulures d'injection.

On a ainsi, par une manipulation simple, pratique et rapide, l'assurance de réaliser des mélanges appropriés aux prescriptions par le choix des poches-doses appropriées.

La possibilité de réaliser des pré-connexions sans communication permet de conditionner des sets de transfert tout prêts qui peuvent être stockés et manipulés facilement, sans risques.

La connexion verrouillée entre les différentes poches évite toute contamination également pendant les opérations d'élimination des poches après usage, puisque l'ensemble est éliminé sans séparation des poches.

Avantageusement, les poches peuvent être constituées de divers matériaux adaptés au contenu spécifique des poches, dans le souci d'éviter d'éventuelles réactions contenant/contenu.

Le dispositif de l'invention est ainsi d'une grande souplesse d'emploi.

D'autres caractéristiques et avantages ressortiront de la description qui va suivre d'un mode de réalisation préféré du set de transfert de l'invention, description donnée à titre d'exemple uniquement et en regard des dessins annexés sur lesquels :
- Figure 1 représente schématiquement un set de transfert selon l'invention ;
- Figure 2a et 2b représentent deux vues en perspectives d'un élément mâle d'un connecteur du type luer conforme à l'invention ;
- Figure 3 est une vue en coupe axiale de l'élément mâle en place à l'extrémité d'une tubulure de liaison ;
- Figure 4 est une vue en élévation latérale d'un élément femelle de connecteur luer associé à l'élément mâle de la figure 3 ;
- Figure 5 est une vue similaire à celle de la figure 4, l'élément femelle ayant subi une rotation autour de son axe de 90°, et
- Figure 6 est une vue en coupe axiale de l'élément femelle de la figure 5.

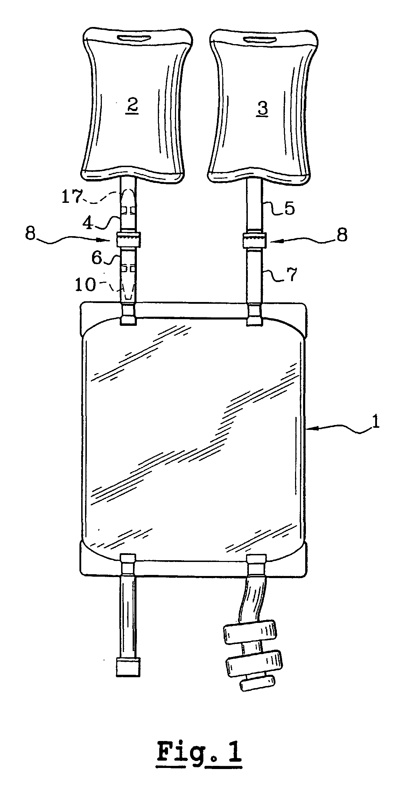

Sur la figure 1, on a représenté en 1 une poche souple dite principale contenant un premier liquide, par exemple un solvant et en 2 et 3, deux petites poches séparées, dites poches auxiliaires, contenant par exemple deux principes actifs de composition différente, destinés à être mélangés au solvant de la poche 1.

Chaque poche 2, 3 comporte une tubulure, respectivement 4 et 5, susceptible de communiquer avec une tubulure, respectivement 6 et 7, reliée à la poche 1, via une connexion de type luer mâle/luer femelle 8, connue en elle-même.

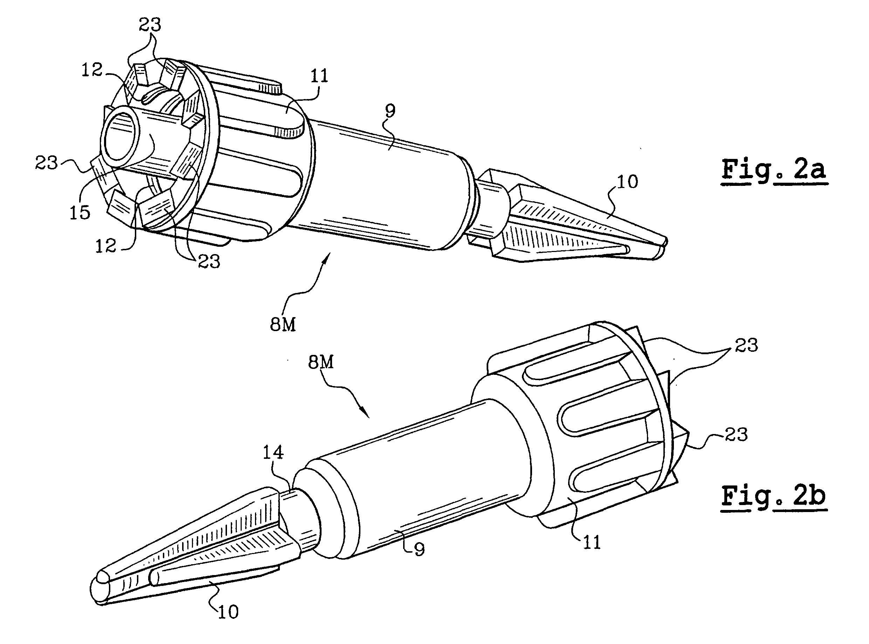

Les figures 2a, 2b, 3 à 6 illustrent un mode de réalisation de deux éléments mâle et femelle conforme à l'invention d'une telle connexion.

Ces éléments comprennent un connecteur de type luer frangible mâle 8m (figures 2a, 2b) et un connecteur de type luer frangible femelle 84 (figure 4, 5) pourvus de moyens d'interconnexion.

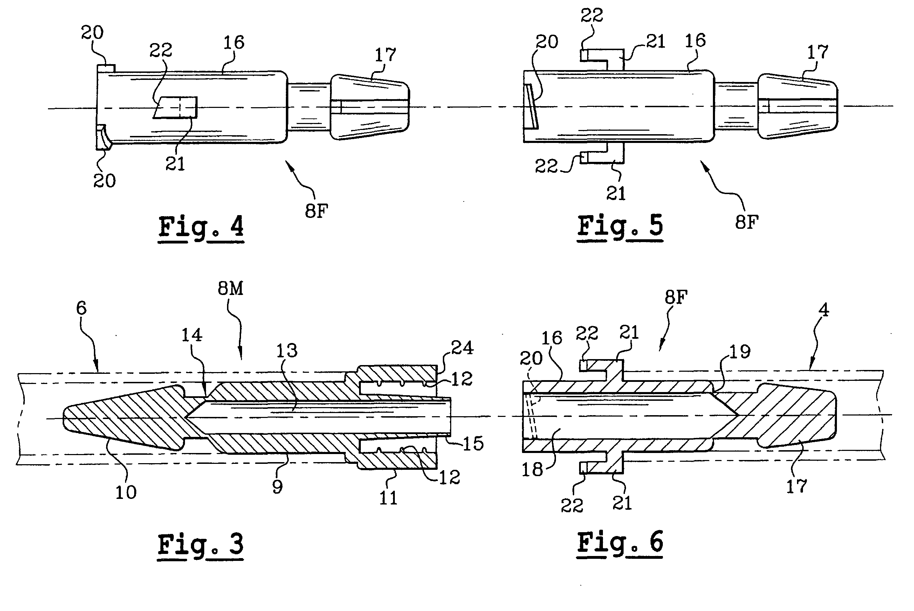

Le connecteur luer mâle 8m comprend un corps cylindrique 9 prolongé, à une extrémité, à la manière connue, par une section frangible 10 de forme conventionnelle, et à l'autre extrémité, par une virole cylindrique d'accouplement 11 munie intérieurement (figure 3) d'un filetage 12.

Le corps 9 est muni d'un passage central 13 se terminant, côté section frangible 10, au voisinage d'un pontage mince 14 où se fera la séparation entre la section 10 et le corps 9, cependant qu'à son autre extrémité, le passage 13 communique avec une partie tubulaire coaxiale 15 faisant légèrement saillie hors de la virole 11.

Comme illustré par la figure 3, le luer mâle 8m est engagé à force dans l'extrémité de la tubulure 6 par exemple, dont l'extrémité vient en butée contre la virole 11 qui présente un diamètre externe sensiblement supérieur à celui du corps 9 et correspondant sensiblement à celui de ladite tubulure 6.

Le connecteur luer femelle 8f comprend un corps cylindrique 16 prolongé, à une extrémité et suivant une caractéristique de l'invention, par une section frangible 17, d'un type analogue à celui de l'élément mâle 8m..

Le corps 16 présente un passage central cylindrique 18 se terminant, côté section frangible 17, au voisinage d'un pontage mince de rupture 19, homologue du pontage 14.

A son autre extrémité, le passage 18 est débouchant et son diamètre correspondant au diamètre externe de la partie tubulaire 15 du luer mâle 8m.

Le luer femelle 8f est susceptible de s'engager dans la virole 11, la partie tubulaire 15 pénétrant dans le passage 18.

Le corps 16 est muni extérieurement à l'opposé de la section frangible 17, de deux sections filetées opposées 20, coopérant avec le filetage 12 de la virole 11, en sorte d'accoupler par vissage les luers mâle 8m et femelle 8f.

Avantageusement, la paroi externe de la partie tubulaire 15 est légèrement conique pour assurer l'étanchéité de la liaison.

Le luer femelle 8f est engagé (figure 6) dans l'extrémité par exemple du tube 4, d'une manière similaire à celle du luer mâle 8m, la section frangible 17 étant à l'intérieur du tube 4 dont l'extrémité, engagée à force sur le corps 16, vient en butée contre deux saillies 21 diamétralement opposées, sur l'extérieur du corps 16.

Les saillies 21 définissant également, du côté tourné vers le luer mâle 8m, deux facettes inclinées 22, susceptibles de coopérer avec des marches inclinées complémentaires 23 ménagées circulairement (figure 2a) sur la tranche 24 de la virole 11, en regard desdites facettes 22.

Les moyens 22, 23 constituent, conformément à l'invention, un dispositif de type cliquet et coopèrent pour, en fin de vissage, constituer un crantage anti-retour interdisant le dévissage des luers assemblés.

Les deux luers 8m, 8f peuvent s'accoupler à tout moment, par exemple pour mettre en place une poche souple dosée 2 sur la poche 1 et préparer ainsi un mélange du contenu de la poche dosée 2 avec celui de la poche 1, lequel mélange pourra être fait ultérieurement à tout moment par simple rupture des pontages 14 et 19 des luers 8m et 8f de la liaison d'aboutage 8.

Les poches 2 et 3 peuvent contenir bien entendu des doses différentes ou non de produits différents ou non.

De préférence, le luer mâle 8m sera engagé dans la tubulure (6, 7) solidaire de la poche 1.

Il est clair qu'une même poche principale 1 peut comporter plusieurs tubulures de transfert telles que 6 ou 7, non raccordées à des poches dosées 2, 3, c'est à dire en attente, ces tubulures étant munies par exemple d'un luer mâle 8m.

N'importe quelle poche dosée comportant une tubulure telle que 4, 5, munie d'un luer femelle 8f peut à tout moment être connectée à la poche 1 et mise en communication avec celle-ci, aussitôt ou plus tard.

On a ainsi l'assurance d'un mélange adéquat de quantités dosées grâce au jeu de poches dosées équipées selon l'invention.

Il est à noter également qu'un set constitué d'une poche principale 1 et d'une ou plusieurs poches auxiliaires dosées 2, 3 peut être ainsi conditionné et stocké sous emballage stérile, les liaisons 8 étant en place avec les sections frangibles 10, 17 bien entendu non rompues.

Le principe du set de transfert selon l'invention donne la possibilité de conditionner le solvant et les principes actifs selon des process industriels complètement disjoints et différents.

A ce propos, les matériaux des diverses poches peuvent être adaptés aux produits pour éviter d'éventuelles réactions contenant/contenu.

Les connexions entre poches présentent une totale sécurité du fait du caractère irréversible de l'assemblage des éléments mâles 8m et femelles 8F, d'où il résulte une asepsie parfaitement contrôlée, tout contact cutané ou aérien du personnel soignant avec des substances potentiellement très actives étant évité.

Toute contamination est également évitée après usage puisque c'est l'ensemble du set qui est évacué, sans séparation des poches.

Le set de transfert de l'invention s'applique d'une manière générale à la réalisation de mélanges dosés notamment dans le domaine de l'administration de substances liquides à des fins nutritionnelles, en chimiothérapie, en antibiothérapie, etc... à partir d'un ensemble de poches souples contenant diverses substances dosées qu'il s'agit de mélanger avant administration.

Enfin, l'invention n'est évidemment pas limitée au mode de réalisation représenté et décrit ci-dessus, mais en couvre au contraire toutes les variantes, notamment en ce qui concerne l'agencement pour effectuer l'accouplement de manière irréversible entre les luers mâle et femelle.

## Revendications

1. Set de transfert notamment pour l'administration d'un mélange de liquides à des fins médicales, à partir d'une poche souple dite principale (1) contenant un premier liquide et d'au moins une poche souple dite auxiliaire (2, 3) contenant un second liquide, **caractérisé en ce que** la poche auxiliaire est raccordable à la poche principale à l'aide d'une paire de connecteurs de type luer respectivement mâle (8 m) et femelle (8 f), chacun des deux connecteurs (8 m, 8 f) comportant, à une extrémité, une section frangible (10, 17) engagée dans l'extrémité d'une tubulure (6, 4) reliée à l'une des poches (1, 2, 3) et, à l'autre extrémité, un moyen (11, 12, 20) d'accouplement à l'autre connecteur, pourvu de moyens (22, 23) de blocage en position accouplée desdits connecteurs (8m, 8f).

2. Set de transfert selon la revendication 1, **caractérisé en ce que** lesdits moyens de blocage sont constitués de moyens de type cliquet anti-retour (22-23) agencés sur les deux parties destinées à s'accoupler (19, 16) des connecteurs (8m, 8f).

3. Set de transfert selon la revendication 1 ou 2, **caractérisé en ce que** la ou les poches auxiliaires (2, 3) sont des poches contenant des quantités dosées de principes actifs destinés à être mélangés à un solvant contenu dans ladite poche principale (1).

## Patentansprüche

1. Übertragungsset insbesondere für die Verabreichung eines Flüssigkeitsgemisches für medizinische Zwecke aus einer nachgiebigen sogenannten Haupttasche (1), die eine erste Flüssigkeit enthält, und aus wenigstens einer weichen sogenannten Hilfstasche (2, 3), die eine zweite Flüssigkeit enthält, **dadurch gekennzeichnet, dass** die Hilfstasche mit der Haupttasche mit Hilfe eines Paars Verbinder des Luer-Typs, einem Steckverbinder (8m) und einem Buchsenverbinder (8f), verbunden werden kann, wobei jeder der zwei Verbinder (8m, 8f) an einem Ende einen zerbrechbaren Abschnitt (10, 17) aufweist, der mit einem Ende eines Rohrs (6, 4) in Eingriff ist, das mit einer der Taschen (1, 2, 3) verbunden ist, und am anderen Ende ein Mittel (11, 12, 20) für die Ankopplung an den anderen Verbinder aufweist, das mit Mitteln (22, 23) zur Verriegelung in der gekoppelten Position der Verbinder (8m, 8f) versehen ist.

2. Übertragungsset nach Anspruch 1, **dadurch gekennzeichnet, dass** die Verriegelungsmittel aus Drehverhinderungsklinkenmitteln (22-23) gebildet sind, die an den beiden Teilen angeordnet sind, die dazu bestimmt sind, an die Verbinder (8m, 8f) angekoppelt (19, 16) zu werden.

3. Übertragungsset nach Anspruch 1 oder 2, **dadurch gekennzeichnet, dass** die Hilfstasche(n) (2, 3) eine Tasche ist bzw. Taschen sind, die abgemessene Mengen aktiver Stoffe enthalten, die dazu bestimmt sind, mit einem in der Haupttasche (1) enthaltenen Lösungsmittel gemischt zu werden.

## Claims

1. A transfer set in particular for administering a mixture of liquids for medical purposes, from a so-called main (1) flexible bag containing a first liquid and at least one so-called auxiliary flexible bag (2, 3) containing a second liquid, **characterised in that** the auxiliary bag can be connected to the main bag by means of a pair of connectors of the Luer type, respectively male (8 m) and female (8 f), each of the two connectors (8 m, 8 f) comprising, at one end, a breakable section (10, 17) engaged in the end of a tube (6, 4) connected to one of the bags (1, 2, 3) and, at the other end, means (11, 12, 20) for coupling to the other connector, provided with means (22, 23) for locking said connectors (8 m, 8 f) in the coupled position.

2. A transfer set according to claim 1, **characterised in that** said locking means are formed of means of the non-return catch type (22-23) arranged on the two parts intended to be coupled (19, 16) to the connectors (8 m, 8 f).

3. A transfer set according to claim 1 or 2, **characterised in that** the auxiliary bag or bags (2, 3) are bags containing an apportioned quantity of active principles intended to be mixed with a solvent contained in said main bag (1).
